# EUROPÄISCHE PATENTSCHRIFT

(11) **EP 1 908 425 B1**
(45) Veröffentlichungstag und Bekanntmachung des Hinweises auf die Patenterteilung: **05.01.2011**
(21) Anmeldenummer: 07015245.9
(22) Anmeldetag: 03.08.2007
(51) Int. Cl.: A61B 17/28

(54) **Medizinisches Rohrschaftinstrument**
Medical tube shaft instrument
Instrument médical à tige cylindrique

(30) Priorität: 17.08.2006 DE 102006038515
(43) Veröffentlichungstag der Anmeldung: 09.04.2008
(73) Patentinhaber: Karl Storz GmbH & Co. KG, 78532 Tuttlingen (DE)
(72) Erfinder: Renger, Uwe, 78247 Hilzingen (DE); Blocher, Martin, 78532 Tuttlingen (DE); Bacher, Uwe, 78532 Tuttlingen (DE)
(74) Vertreter: Hofmeister, Frank

(56) Entgegenhaltungen:
- DE-A1-102004 025 041
- US-A- 5 653 713

## Beschreibung

Die Erfindung betrifft ein medizinisches Rohrschaftinstrument mit einem hohlen Schaft, einer am proximalem Ende des Schaftes angeordneten, mit mindestens zwei Griffteilen versehenen Handhabe und mindestens einer in dem hohlen Schaft gelagerten Zug-/Schubstange, an deren distalem Ende ein aus mindestens zwei Maulteilen bestehendes Werkzeug angeordnet ist, wobei die Zug-/Schubstange zum Öffnen und Schließen mindestens eines Maulteils des Werkzeugs mit mindestens einem verschwenkbaren Griffteil der Handhabe koppelbar ist und wobei die Zug-/Schubstange und die Handhabe über einen Kopplungsmechanismus lösbar miteinander verbindbar sind, der als mindestens eine Spannbacke aufweisende Klemmvorrichtung ausgebildet ist.

Derartige medizinische Rohrschaftinstrumente finden beispielsweise in der Ausbildung als Nadelhalter Verwendung in der endoskopischen Chirurgie. Aufgrund steigender hygienischer Anforderungen wird immer häufiger gefordert, dass insbesondere Hohlräume, wie beispielsweise hohle Schäfte; aufweisende Rohrschaftinstrumente zumindest teilweise zerlegbar ausgebildet sind, um diese einer gründlichen Reinigung und Sterilisation, vorzugsweise Dampfsterilisation, unterziehen zu können.

Aus der DE 43 07 539 A1 ist ein gattungsgemäßes, als medizinische Zange ausgebildetes medizinisches Rohrschaftinstrument bekannt. Dieses bekannte Rohrschaftinstrument lässt sich zum Reinigen und Sterilisieren in drei Hauptgruppen zerlegen, die Zug-/Schubstange, den hohlen Schaft sowie die Handhabe. Der Kopplungsmechanismus zum Verbinden der Zug-/Schubstange mit der Handhabe besteht bei dieser Konstruktion aus einer am proximalen Ende der Zug-/Schubstange angeordneten Kupplungskugel, die in eine korrespondierend geformte Kupplungsaufnahme im verschwenkbaren Griffteil einzusetzen ist. Zwar hat sich diese Kupplung in der Praxis bewährt, jedoch bedarf diese Kupplung einer sehr exakten Fertigung der Zug-/Schubstange. DE 10 2004 025 041 beschreibt ein Instrument gemäß Präambel des Anspruchs 1.

Davon ausgehend liegt der Erfindung die Aufgabe zugrunde, ein medizinisches Rohrschaftinstrument derart weiterzubilden, dass die Zug-/Schubstange und die Handhabe über einen im Wesentlichen spielfreien und einfach zu fertigenden Kopplungsmechanismus lösbar miteinander verbindbar sind.

Die Lösung dieser Aufgabenstellung ist erfindungsgemäß in den kennzeichnenden Teil des Anspruchs 1 zu finden.

Aufgrund dieser Ausgestaltung des Kopplungsmechanismus als eine mit mindestens eine in einer Führungsbahn gelagerte Spannbacke wird eine im Wesentlichen spielfreie Kopplung zwischen der Zug-/Schubstange und der Handhabe sichergestellt, die auch Längenabweichungen der Zug-/Schubstange toleriert.

Durch die Lagerung der Spannbacke in der Führungsbahn wird das Verschwenken der mindestens einen Spannbacke zwischen der geschlossen Klemmposition und der offenen Montageposition erleichtert, wobei durch die Ausbildung der Führung für die Spannbackenverstellung eine exakte und gleichmäßige Überführung der mindestens einen Spannbacke in die jeweilige Kopplungsposition gewährleistet wird. Erfindungsgemäß ist an jeder in einer Führungsbahn gelagerten Spannbacke ein in die Führungsbahn eingreifender Steuerzapfen angeordnet.

Gemäß einer bevorzugten Ausführungsform der Erfindung weist die Klemmvorrichtung zwei Spannbacken auf.

Gemäß einer praktischen Ausführungsform der Erfindung wird vorgeschlagen, dass die Spannbacken der Klemmvorrichtung das proximale Ende der Zug-/Schubstange zumindest teilweise formschlüssig umgreifen, wobei das proximale Ende der Zug-/Schubstange vorzugsweise als von den Spannbacken zumindest teilweise umgreifbares Kopplungselement ausgebildet ist. Die Ausbildung der das proximale Ende der Zug-/Schubstange ergreifenden Spannbacken ermöglicht eine platzsparende Bauweise des Kopplungsmechanismus bei gleichzeitig guter Kraftübertragung aufgrund der großen Übertragungsfläche.

Zur Ausbildung der Klemmverbindung zwischen den Spannbacken und der Zug-/Schubstange ist das Kopplungselement erfindungsgemäß als eine in Axialrichtung der durch die Spannbacken gebildeten Einspannstelle nachgelagerte Erweiterung der Zug-/Schubstange ausgebildet. Solchermaßen ausgebildet ist es möglich, die Zug-/Schubstange im Bereich der Einspannstelle form- oder reibschlüssig, in jedem fall aber klemmend mittels der Spannbacken festzulegen.

Das Kopplungselement ist gemäß einer praktischen Ausführungsform der Erfindung durch eine in Axialrichtung dem proximalen Ende vorgelagerte Einschnürung der Zug-/Schubstange ausgebildet.

Alternativ wird gemäß einer zweiten Ausgestaltungsform der Erfindung vorgeschlagen, dass zumindest eine der Spannbacken als Federelement ausgebildet ist, wobei die Federelastizität dieser mindestens einen Spannbacke nicht nur durch die Eigenelastizität des Spannbackenwerkstoffs, sondern auch die Federbelastung dieser mindestens einen Spannbacke erzielbar ist.

Das Einführen des proximalen Endes der Zug-/Schubstange in die Klemmvorrichtung kann erfindungsgemäß dadurch erleichtert werden, dass am Kopplungselement und/oder an den Spannbacken ein Verkanten der gegeneinander anlaufenden Bauteile verhindernde Anlaufschrägen ausgebildet sind.

Weiterhin wird mit der Erfindung vorgeschlagen, dass die Spannbacken verschwenkbar an einem mit dem mindestens einen verschwenkbaren Griffteil der Handhabe koppelbaren Verbindungsglied gelagert sind.

Schließlich wird mit der Erfindung vorgeschlagen, dass die Klemmvorrichtung über ein Federelement in die die Spannbacken öffnende Stellung vorgespannt ist, um ein einfaches Lösen der Kupplung zu ermöglichen.

Weitere Merkmale und Vorteile der Erfindung ergeben sich anhand der zugehörigen Zeichnung, in der ein Ausführungsbeispiel eines erfindungsgemäßen medizinischen Rohrschaftinstruments nur beispielhaft dargestellt ist. In der Zeichnung zeigt:
- Fig. 1: eine schematische Seitenansicht eines erfindungsgemäßen Rohrschaft- instruments;
- Fig. 2: eine schematische Seitenansicht des Instruments gemäß Fig. 1 im zer- legten Zustand;
- Fig. 3: eine vergrößerte ausschnittweise schematische Schnittzeichnung des Details III gemäß Fig. 1;
- Fig. 4a: eine vergrößerte ausschnittweise schematische Darstellung gemäß Fig. 3, jedoch die Klemmvorrichtung in der geöffneten Position darstellend und
- Fig. 4b: eine Darstellung gemäß Fig. 4a, jedoch die Klemmvorrichtung in der geschlossenen Position darstellend.

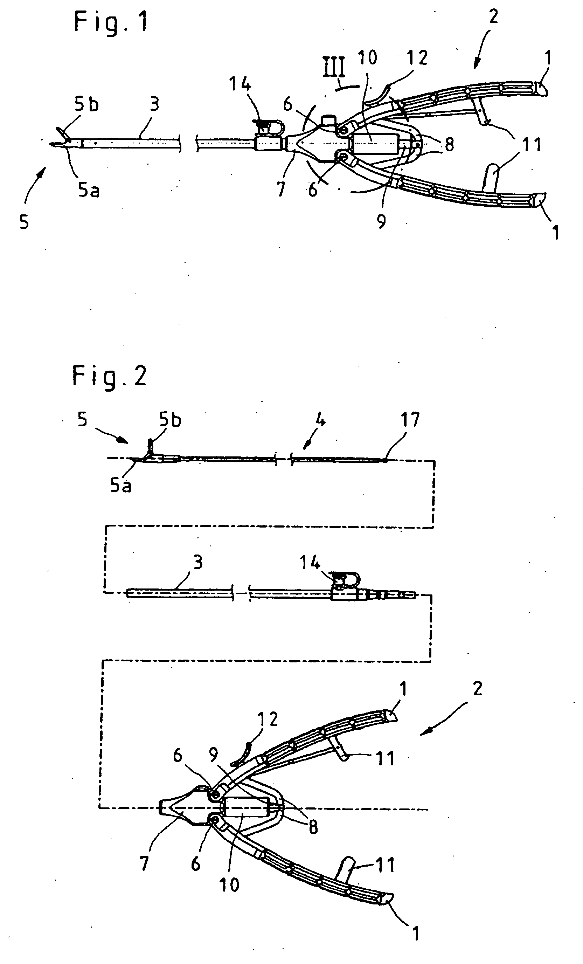

Das in den Abbildungen Fig. 1 und 2 dargestellte, als Nadelhalter ausgebildete medizinische Rohrschaftinstrument besteht im Wesentlichen aus einer mit zwei Griffteilen 1 versehenen Handhabe 2, einem hohlen Schaft 3 sowie einer in den hohlen Schaft 3 einsetzbaren Zug-/Schubstange 4, an deren distalem Ende ein aus zwei Maulteilen 5a und 5b bestehendes Werkzeug 5 angeordnet ist.

Die in Fig. 2 besonders deutlich dargestellten Baugruppen Handhabe 2, hohler Schaft 3 und Zug-/Schubstange 4 sind über Kopplungs- und Rastmechanismen so miteinander koppelbar, dass durch Betätigen der Griffteile 1 der Handhabe 2 die Maulteile 5a und 5b des Werkzeugs 5 zwischen einer offenen und einer geschlossenen Arbeitsstellung verstellbar sind, wobei die vom Benutzer auf die Griffteile 1 der Handhabe 2 aufgebrachten Kräfte über die Zug-/Schubstange 4 auf die Maulteile 5a, 5b des Werkzeugs 5 übertragen werden.

Bei der dargestellten Ausführungsform weist das Werkzeug 5 ein starres Maulteil 5a und ein gegenüber dem starren Maulteil 5a verschwenkbares Maulteil 5b auf. Selbstverständlich ist es auch möglich, beide Maulteile des Werkzeugs 5 als verschwenkbare Maulteile 5b auszubilden.

Wie aus Fig. 1 und 2 ersichtlich, sind bei der dargestellten Ausführungsform beide Griffteile 1 der Handhabe 2 als verschwenkbare Griffteile 1 ausgebildet, die über Anlenkpunkte 6 verschwenkbar an einem Gehäuse 7 der Handhabe 2 gelagert sind. Zur Überführung der Schwenkbewegung der Griffteile 1 in eine reine Axialbewegung der Zug-/Schubstange 4 sowie zur Kraftübertragung der vom Benutzer über die Handhabe 2 eingeleiteten Druckkraft auf die Zug-/Schubstange 4 sind beide Griffteile 1 über jeweils einen Gelenkhebel 8 mit einer Kupplungsstange 9 verbunden, die ihrerseits direkt oder indirekt über einen Kopplungsmechanismus mit der Zug-/Schubstange 4 gekoppelt ist, wobei die Kopplung der Zug-/Schubstange 4 mit der Kupplungsstange 9 und somit mit der Handhabe 2 in dem Kupplungsgehäuse 10 erfolgt.

Die Kopplung der Zug-/Schubstange 4 und somit auch der Maulteile 5a und 5b des Werkzeugs 5 mit den Griffteilen 1 der Handhabe 2 ist so ausgelegt, dass beim Zusammendrücken der Griffteile 1 die Zug-/Schubstange 4 über die Gelenkhebel 8 und die Kupplungsstange 9 in axialer Richtung zum proximalen Ende des Instruments gezogen wird. Diese Axialverschiebung der Zug-/Schubstange 4 zum proximalen Ende des Instruments bewirkt ein Überführen der Maulteile 5a, 5b des Werkzeugs 5 in die geschlossene Arbeitsstellung. In dieser zusammengedrückten Stellung sind die Griffteile 1 über eine Arretiervorrichtung 11 gegeneinander fixierbar, so dass der Benutzer nicht die gesamte Zeit die Druckkraft auf die Griffteile 1 der Handhabe 2 ausüben muss. Über einen Entriegelungsknopf 12, der die Teile der Arretiervorrichtung 11 trennt, ist diese Fixierung wieder aufhebbar.

Alternativ zu der dargestellten Ausführungsform ist es selbstverständlich auch möglich, dass die Kopplung der Zug-/Schubstange 4 und somit auch der Maulteile 5a und 5b des Werkzeugs 5 mit den Griffteilen 1 der Handhabe 2 so ausgelegt ist, dass die Zug-/Schubstange 4 beim Zusammendrücken der Griffteile 1 in axialer Richtung zum distalen Ende des Instruments geschoben wird.

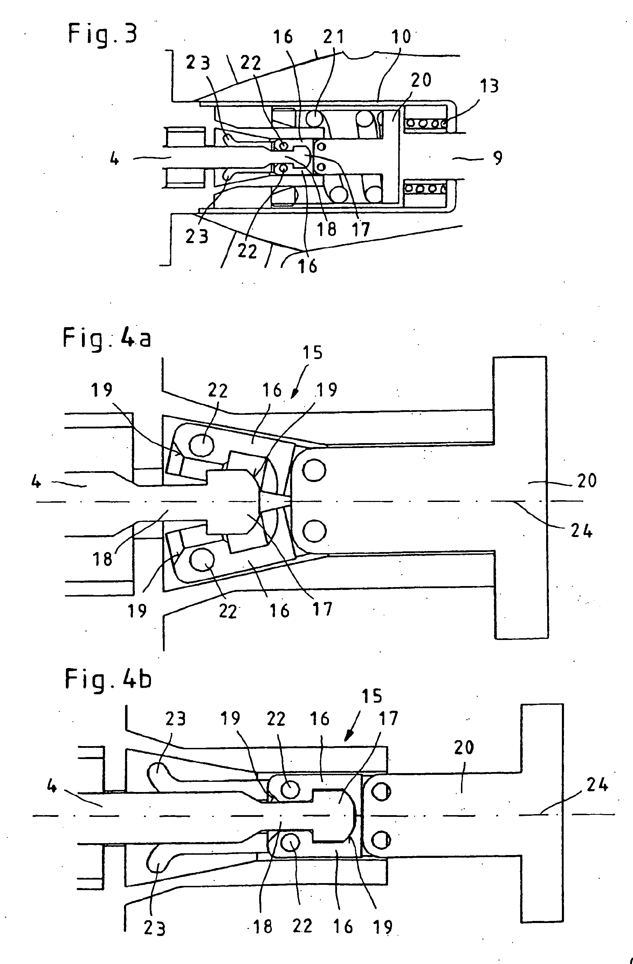

Vorteilhafterweise sind die Griffteile 1 über ein Federelement 13 in die Offenstellung vorgespannt, das, wie beispielsweise in Fig. 3 dargestellt, im Kupplungsgehäuse 10 angeordnet ist. Sobald der Entriegelungsknopf 12 betätigt wird, schiebt dann dieses Federelement 13 die Zug-/Schubstange 4 in axialer Richtung zum distalen Ende des Instruments, wodurch die Griffteile 1 über die Kupplungsstange 9 und die Gelenkhebel 8 auseinander gedrückt werden. Diese Axialverschiebung der Zug-/Schubstange 4 zum distalen Ende des Instruments bewirkt ein Überführen der Maulteile 5a, 5b des Werkzeugs 5 in die offene Arbeitsstellung.

Alternativ zu der dargestellten Ausführungsform der Handhabe 2 mit zwei verschwenkbaren Griffteilen 1 ist es selbstverständlich auch möglich, nur ein Griffteil 1 verschwenkbar auszubilden, wohingegen das andere Griffteil dann beispielsweise einstückig starr mit dem Gehäuse 7 der Handhabe 2 ausgebildet ist. Bei einer solchen Ausgestaltung ist es möglich, die Zug-/Schubstange 4 direkt mit dem verschwenkbaren Griffteil 1 zu koppeln.

Der zur Aufnahme der Zug-/Schubstange 4 dienende hohle Schaft 3 ist über einen Kopplungs- oder Rastmechanismus mit der Handhabe 2 koppelbar, der im Gehäuse 7 der Handhabe 2 angeordnet ist. Bei der dargestellten Ausführungsform des medizinischen Rohrschaftinstruments weist der hohle Schaft 3 weiterhin einen Spülanschluss 14 auf, der einerseits zum Einleiten von Spülflüssigkeit während einer Operation dient und an den andererseits zum Reinigen des hohlen Schafts 3 ein Spülschlauch anschließbar ist.

Auch distalseitig weist der hohle Schaft 3 einen Kopplungs- oder Rastmechanismus auf, um den hohlen Schaft 3 und die in diesen einsetzbare Zug-/Schubstange 4 miteinander koppeln zu können.

Der Aufbau des im Kupplungsgehäuse 10 angeordneten Kopplungsmechanismus zum lösbaren Verbinden der Zug-/Schubstange 4 mit der Handhabe 2 ist den Abbildungen Fig. 3 bis 4b zu entnehmen.

Dieser Kopplungsmechanismus ist als Klemmvorrichtung 15 ausgebildet, die bei der dargestellten Ausführungsform aus zwei verschwenkbar gelagerten Spannbacken 16 besteht, die das proximale Ende der Zug-/Schubstange 4 zumindest teilweise formschlüssig umgreifen. Zur Aufnahme in den Spannbacken 16 der Klemmvorrichtung 15 ist am proximalen Ende der Zug-/Schubstange 4 ein Kopplungselement 17 ausgebildet, das bei der dargestellten Ausgestaltungsform als durch eine in Axialrichtung dem proximalen Ende vorgelagerte Einschnürung 18 hammerkopfförmig ausgebildet ist.

Um das Einführen des Kopplungselements 17 in die Spannbacken 16 zu erleichtern, sind an den gegeneinander anlaufenden Stirnflächen sowohl des Kopplungselements 17 als auch der Spannbacken 16 Anlaufschrägen 19 ausgebildet, durch die der Reibungswiderstand der gegeneinander anlaufenden Bauteile herabgesetzt wird.

Wie aus den Abbildungen Fig. 3 bis 4b ersichtlich, sind die beiden Spannbacken 16 mit ihrer proximalen Enden verschwenkbar an der distalen Seite eines Verbindungsglieds 20 gelagert, das über ein Federelement 21 an der Kupplungsstange 9 anliegt, die ihrerseits über die Gelenkhebel 8 mit den Griffteilen 1 gekoppelt ist. Durch das Betätigen der Griffteile 1 der Handhabe 2 können somit die Spannbacken 16 der Klemmvorrichtung 15 im Wesentlichen spielfrei betätigt werden, wobei das Koppeln der Zug-/Schubstange 4 mit den Spannbacken 16 ausschließlich in der in Fig. 4a dargestellten Montagestellung mit geöffneten Spannbacken 16 erfolgen kann.

Bei dem im Kopplungsbereich der Zug-/Schubstange 4 mit der Handhabe 2 angeordnete Federelement 21 handelt es sich um eine als Überlastfeder 21 ausgebildete Überlastsicherung, die eine zu große Krafteinleitung in die Zug-/Schubstange 4 verhindert. Wenn bei bereits geschlossenen Maulteilen 5a und 5b des Werkzeugs 5 die Griffteile 1 der Handhabe 2 weiter zusammengedrückt werden, wird diese über die Kopplungsstange 9 und das Verbindungsglied 20 auf die Zug-/Schubstange 4 zu übertragende Zugkraft von der Überlastfeder 21 aufgefangen und verhindert so eine Beschädigung der Zug-/Schubstange 4 und/oder der Maulteile 5a und 5b des Werkzeugs 5. Ebenso verhindert die Überlastsicherung bei Sicherung der durch die Spannbacken 16 bewirkten Klemmverbindung eine mögliche Beschädigung des Verbindungsglieds 20 sowie der Spannbacken 16.

Distalseitig sind die Spannbacken 16 über jeweils einen Steuerzapfen 22 in einer Führungsbahn 23 gelagert. Über diese Zapfen-Schlitz-Steuerung werden die Spannbacken 16 bei einer Axialverschiebung der Klemmvorrichtung 15 zwischen der offenen Montagestellung (Fig. 4a) und der geschlossenen Klemmstellung (Fig,. 4b) verstellt. Da, wie aus den Abbildungen Fig. 3 und 4b ersichtlich, die Führungsbahnen 23 nur an ihren distalseitigen Enden radial nach außen verlaufend abgewinkelt sind, ansonsten aber parallel zur Instrumentenlängsachse 24 verlaufen, können die Spannbacken 16 nur dann geöffnet werden, wenn die die Spannbacken 16 führenden Steuerzapfen 22 in diese distalseitigen Abwinklungen der Führungsbahnen 23 eintreten und somit die Spannbacken 16 in die geöffnete Montageposition verschwenken.

Alternativ zu der dargestellten Ausgestaltungsform der Spannbacken 16 ist es selbstverständlich auch möglich, diese als Federelemente oder federelastische Spannbacken 16 auszugestalten, die durch Eigenspannung oder eine externe Federkraft in die Schließstellung vorgespannt sind und so das proximale Ende der Zug-/Schubstange 4 formschlüssig klemmend umgreifen.

Das Zusammensetzen des dargestellten Rohrschaftinstruments und insbesondere des Kopplungsmechanismus zum Verbinden des hohlen Schaftes 3 mit der Zug-/Schubstange 4 wird nachfolgend anhand der Abbildungen Fig. 4a und 4b beschrieben.

In den ersten beiden Montageschritten werden die Zug-/Schubstange 4 und der hohle Schaft 3 miteinander gekoppelt und der hohle Schaft 3 mit der Handhabe 2 verbunden.

Nachfolgend werden die Griffteile 1 der Handhabe 2 so weit wie möglich auseinander in die Offenstellung gedrückt, worin sie durch das im Kupplungsgehäuse 10 angeordnete Federelement 13 unterstützt werden. Das Auseinanderdrücken der Griffteile 1 der Handhabe 2 bewirkt über die Gelenkhebel 8 eine Axialverschiebung der Kupplungsstange 9 in Richtung des distalen Endes des Rohrschaftinstruments. Durch die direkte Kopplung der Kupplungsstange 9 mit dem Verbindungsglied 20, an dem die Spannbacken 16 gelagert sind, bewirkt das Auseinanderdrücken der Griffteile 1 der Handhabe 2 weiterhin eine Verschiebung der Spannbacken 16 in distale Richtung. Durch die Zapfen-Schlitz-Steuerung der in die Führungsbahnen 23 eingreifenden Steuerzapfen 22 werden die Spannbacken 16 bei dieser Axialverschiebung in die in Fig. 4a dargestellte offene Montagestellung überführt.

In dieser Stellung kann nun die Zug-/Schubstange 4 mit dem als Kupplungselement 17 ausgebildeten proximalen Ende voran in die geöffneten Spannbacken 16 eingeführt werden, was durch die beidseitig ausgebildeten Anlaufschrägen 19 erleichtert wird. In dieser offenen Montagestellung nehmen die Griffteile 1 der Handhabe 2 eine über-offene Stellung ein, die diese bei der praktischen Handhabung des Instruments nie einnehmen können. Diese über-offene Stellung der Griffteile 1 dient alleine zur Montage der Zug-/Schubstange 4, wodurch ein versehentliches Lösen der Zug-/Schubstange 4 im normalen Betrieb ausgeschlossen werden kann.

Um ein versehentliches Überführen der Griffteile 1 der Handhabe 2 und somit auch des Werkzeugs 5 in die über-offene Montagestellung zu verhindern, ist es möglich, beispielsweise im Bereich des in der Handhabe 2 gelagerten proximalen Endes der Zug-/Schubstange 4 und des hohlen Schaftes 3 eine die Verlagerbarkeit der Zug-/Schubstange 4 innerhalb des hohlen Schaftes 3 limitierende Begrenzungsvorrichtung anzuordnen. Diese nur im fertig montierten Zustand des Rohrschaftinstruments wirksame Begrenzungsvorrichtung erlaubt einen Axialhub der Zug-/Schubstange 4 nur innerhalb der begrenzten Wegstrecke, die notwendig ist, um die Maulteile 5a, 5b des Werkzeugs 5 zwischen der in Fig. 1 dargestellten offenen Arbeitsstellung und der geschlossenen Arbeitsstellung zu verschwenken. Aufgrund der Kopplung der Axialverlagerung der Zug-/Schubstange 4 mit dem Verschwenkwinkel der Griffteile 1 der Handhabe 2 bewirkt somit die den Axialhub der Zug-/Schubstange 4 beschränkende Begrenzungsvorrichtung gleichzeitig eine Limitierung des Verschwenkwinkel der Griffteile 1 im fertig montierten Zustand.

Zum Abschluss der Montage wird die Zug-/Schubstange 4 weiter in Axialrichtung hin zum proximalen Ende des Rohrschaftinstruments gedrückt, was durch das Zusammendrücken der Griffteile 1 der Handhabe 2 unterstützt werden kann. Bei dieser Axialverschiebung der Zug-/Schubstange 4 in Richtung des proximalen Endes des Rohrschaftinstruments werden die Spannbacken 16 entlang der Führungsbahnen 23 nach proximal verlagert, wodurch diese in die geschlossene, das proximale Ende der Zug-/Schubstange 4 formschlüssig umgreifende Stellung überführt werden. In dieser Stellung bewirkt die Klemmvorrichtung 15 eine spielfreie und zuverlässige Kopplung der Zug-/Schubstange 4 mit der Handhabe 2.

Die Demontage erfolgt dann in genau umgekehrter Reihenfolge der Montageschritte über das Lösen der Verrastung zwischen dem hohlen Schaft 3 und der Handhabe 2, das Lösen der Verbindung der Zug-/Schubstange 4 mit den Griffteilen 1 der Handhabe 2 bis hin zum Entkoppeln des hohlen Schaftes 3 von der Zug-/Schubstange 4. Zum Lösen der Verbindung der Zug-/Schubstange 4 mit den Griffteilen 1 der Handhabe 2 werden die Griffteile 1 wieder so weit wie möglich auseinander gedrückt, bis die Spannbacken 16 wieder die in Fig. 4a dargestellte Montagestellung eingenommen haben und nachfolgend die Zug-/Schubstange 4 von der Handhabe 2 entkoppelt werden kann.

### Bezugszeichenliste

- 1: Griffteil
- 2: Handhabe
- 3: hohler Schaft
- 4: Zug-/Schubstange
- 5: Werkzeug
- 5a: starres Maulteil
- 5b: verschwenkbares Maulteil
- 6: Anlenkpunkt
- 7: Gehäuse
- 8: Gelenkhebel
- 9: Kupplungsstange
- 10: Kupplungsgehäuse
- 11: Arretiervorrichtung
- 12: Entriegelungsknopf
- 13: Federelement
- 14: Spülanschluss
- 15: Klemmvorrichtung
- 16: Spannbacke
- 17: Kopplungselement
- 18: Einschnürung
- 19: Anlaufschräge
- 20: Verbindungsglied
- 21: Federelement/Überlastfeder
- 22: Steuerzapfen
- 23: Führungsbahn
- 24: Längsachse

## Patentansprüche

1. Medizinisches Rohrschaftinstrument mit einem hohlen Schaft (3), einer am proximalem Ende des Schaftes (3) angeordneten, mit mindestens zwei Griffteilen (1) versehenen Handhabe (2) und mindestens einer in dem hohlen Schaft (3) gelagerten Zug-/Schubstange (4), an deren distalem Ende ein aus mindestens zwei Maulteilen (5a, 5b) bestehendes Werkzeug (5) angeordnet ist, wobei die Zug-/Schubstange (4) zum Öffnen und Schließen mindestens eines Maulteils (5b) des Werkzeugs (5) mit mindestens einem verschwenkbaren Griffteil (1) der Handhabe (2) koppelbar ist und wobei die Zug-/Schubstange (4) und die Handhabe (2) über einen Kopplungsmechanismus lösbar miteinander verbindbar sind, der als mindestens eine Spannbacke (16) aufweisende Klemmvorrichtung (15) ausgebildet ist, wobei
die mindestens eine Spannbacke (16) in einer Führungsbahn (23) gelagert zwischen einer geschlossen Klemmposition und einer offenen Montageposition verschwenkbar ist, **dadurch gekennzeichnet, dass** an jeder in einer Führungsbahn (23) gelagerten Spannbacke (16) ein in die Führungsbahn (23) eingreifender Steuerzapfen (22) angeordnet ist.

2. Medizinisches Rohrschaftinstrument nach Anspruch 1, **dadurch gekennzeichnet, dass** die Klemmvorrichtung (15) zwei Spannbacken (16) aufweist.

3. Medizinisches Rohrschaftinstrument nach Anspruch 1 oder 2, **dadurch gekennzeichnet, dass** die Spannbacken (16) der Klemmvorrichtung (15) das proximale Ende der Zug-/Schubstange (4) zumindest teilweise formschlüssig umgreifen.

4. Medizinisches Rohrschaftinstrument nach Anspruch 3, **dadurch gekennzeichnet, dass** das proximale Ende der Zug-/Schubstange (4) als von den Spannbacken (16) zumindest teilweise umgreifbares Kopplungselement (17) ausgebildet ist.

5. Medizinisches Rohrschaftinstrument nach Anspruch 4, **dadurch gekennzeichnet, dass** das Kopplungselement (17) als eine in Axialrichtung der durch die Spannbacken (16) gebildete Einspannstelle nachgelagerte Erweiterung der Zug-/Schubstange (4) ausgebildet ist.

6. Medizinisches Rohrschaftinstrument nach Anspruch 4, **dadurch gekennzeichnet, dass** das Kopplungselement (17) durch eine in Axialrichtung dem proximalen Ende vorgelagerte Einschnürung (18) der Zug-/Schubstange (4) ausgebildet ist.

7. Medizinisches Rohrschaftinstrument nach einem der Ansprüche 4 bis 6, **dadurch gekennzeichnet, dass** zumindest eine der Spannbacken (16) als Federelement ausgebildet ist.

8. Medizinisches Rohrschaftinstrument nach einem der Ansprüche 4 bis 7, **dadurch gekennzeichnet, dass** am Kopplungselement (17) und/oder an den Spannbacken (16) Anlaufschrägen (19) ausgebildet sind.

9. Medizinisches Rohrschaftinstrument nach einem der Ansprüche 1 bis 8, **dadurch gekennzeichnet, dass** die Spannbacken (16) verschwenkbar an einem mit dem mindestens einen verschwenkbaren Griffteil (1) der Handhabe (2) koppelbaren Verbindungsglied (20) gelagert sind.

10. Medizinisches Rohrschaftinstrument nach einem der Ansprüche 1 bis 9, **dadurch gekennzeichnet, dass** die Klemmvorrichtung (15) über ein Federelement (13) in die die Spannbacken (16) öffnende Stellung vorgespannt ist.

## Claims

1. Medical tubular shank instrument with a hollow shank (3), a handle (2) located at the proximal end of the shank (3) provided with at least two handle components (1) and at least one pull/push rod (4) mounted in the hollow shank (3), at the distal end of which a tool (5) is mounted consisting of at least two jaw parts (5a, 5b), whereby the pull/push rod (4) for opening and closing of at least one jaw part (5b) of tool (5) can be coupled with at least one rotatable handle component (1) of handle (2), and whereby the pull/push rod (4) and the handle (2) can be connected detachable with each other by a coupling mechanism, that is designed as clamping device (15) that has at least one collet (16), whereby the at least one collet (16) is mounted in a guide track (23) and can be rotated between a closed clamping position and an open assembly position,
**characterized by,**
that at each collet (16) mounted in a guide track (23), a control pin (22) is mounted that engages with the guide track (23).

2. Medical tubular shank instrument according to Claim 1, **characterized by**, that the clamping device (15) is provided with two collets (16).

3. Medical tubular shank instrument according to claim 1 or 2, **characterized by**, that the collets (16) of the clamping device (15) enclose the proximal end of the pull/push rod (4) form-fit at least in sections.

4. Medical tubular shank instrument according to Claim 3, **characterized by**, that the proximal end of the pull/push rod (4) is designed as coupling element (17) that can at least be partially encompassed by collets (16).

5. Medical tubular shank instrument according to Claim 4, **characterized by**, that the coupling element (17) is designed as the expansion of the pull/push rod (4) in the axial direction downstream of the fixing point formed by the collets (16).

6. Medical tubular shank instrument according to Claim 4, **characterized by**, that the coupling element (17) is designed as a constriction (18) of the pull/push rod (4) upstream of the proximal end in the axial direction.

7. Medical tubular shank instrument according to one of claims 4 to 6, **characterized by**, that at least one of the collets (16) is designed as spring element.

8. Medical tubular shank instrument according to one of claims 4 to 7, **characterized by**, that at the coupling element (17) and/or at the collets (19), start-up chamfers (19) are formed.

9. Medical tubular shank instrument according to one of claims 1 to 8, **characterized by**, that the collets (16) are mounted rotatable on a connection element (20) that can be coupled with the at least one rotatable handle component (1) of handle (2).

10. Medical tubular shank instrument according to one of claims 1 to 9, **characterized by**, that the clamping device (15) is pre-loaded into the position that opens the collets (16) by a spring element (13).

## Revendications

1. Instrument médical à corps allongé tubulaire comprenant un corps allongé creux (3), une poignée de manoeuvre (2) agencée à l'extrémité proximale du corps allongé creux (3) et dotée d'au moins deux branches de préhension (1), et au moins une tige de traction/poussée (4) montée dans le corps allongé creux (3) et à l'extrémité distale de laquelle est agencé un outil (5) constitué d'au moins deux pièces de mâchoire (5a, 5b), la tige de traction/poussée (4) pouvant, pour l'ouverture et la fermeture d'au moins une pièce de mâchoire (5b) de l'outil (5), être couplée à au moins une branche de préhension pivotante (1) de la poignée de manoeuvre (2), et la tige de traction/poussée (4) et la poignée de manoeuvre (2) pouvant être reliées l'une à l'autre de manière amovible par l'intermédiaire d'un mécanisme de couplage, qui est réalisé en tant que dispositif de serrage (15) présentant au moins un mors de serrage (16), ledit au moins un mors de serrage (16), monté dans une voie de guidage (23), pouvant pivoter entre une position fermée de serrage et une position ouverte de montage,
**caractérisé en ce que** sur chaque mors de serrage (16) monté dans une voie de guidage (23), est agencé un tenon de commande (22) venant en prise dans la voie de guidage (23).

2. Instrument médical à corps allongé tubulaire selon la revendication 1, **caractérisé en ce que** le dispositif de serrage (15) comporte deux mors de serrage (16).

3. Instrument médical à corps allongé tubulaire selon la revendication 1 ou la revendication 2, **caractérisé en ce que** les mors de serrage (16) du dispositif de serrage (15) entourent au moins en partie l'extrémité proximale de la tige de traction/poussée (4) par complémentarité de formes.

4. Instrument médical à corps allongé tubulaire selon la revendication 3, **caractérisé en ce que** l'extrémité proximale de la tige de traction/poussée (4) est réalisée en tant qu'élément de couplage (17) pouvant être entouré au moins partiellement par les mors de serrage (16).

5. Instrument médical à corps allongé tubulaire selon la revendication 4, **caractérisé en ce que** l'élément de couplage (17) est réalisé par un évasement de la tige de traction/poussée (4), qui est situé, dans la direction axiale, après la zone de serrage formée par les mors de serrage (16).

6. Instrument médical à corps allongé tubulaire selon la revendication 4, **caractérisé en ce que** l'élément de couplage (17) est réalisé par un rétrécissement (18) de la tige de traction/poussée (4), qui est situé, dans la direction axiale, avant l'extrémité proximale.

7. Instrument médical à corps allongé tubulaire selon l'une des revendications 4 à 6, **caractérisé en ce que** l'un au moins des mors de serrage (16) est réalisé sous forme d'élément élastique.

8. Instrument médical à corps allongé tubulaire selon l'une des revendications 4 à 7, **caractérisé en ce que** des rampes inclinées d'entrée en contact (19) sont formées sur l'élément de couplage (17) et/ou sur les mors de serrage (16).

9. Instrument médical à corps allongé tubulaire selon l'une des revendications 1 à 8, **caractérisé en ce que** les mors de serrage (16) sont montés pivotants sur un organe de liaison (20) pouvant être couplé à ladite au moins une branche de préhension (1) de la poignée de manoeuvre (2).

10. Instrument médical à corps allongé tubulaire selon l'une des revendications 1 à 9, **caractérisé en ce que** le dispositif de serrage (15) est précontraint, par l'intermédiaire d'un élément de ressort (13), dans la position ouvrant les mors de serrage (16).
